# EUROPÄISCHE PATENTSCHRIFT

(11) **EP 0 772 633 B1**
(45) Veröffentlichungstag und Bekanntmachung des Hinweises auf die Patenterteilung: **14.05.2003**
(21) Anmeldenummer: 95923183.8
(22) Anmeldetag: 21.06.1995
(51) Int. Cl.: C07K 14/575, A61K 51/08

(54) **KOMPLEXVERBINDUNGEN ZUR DIAGNOSE VON GEFÄSSERKRANKUNGEN**
COMPLEXES FOR USE IN THE DIAGNOSIS OF VASCULAR DISEASES
COMPLEXES DESTINES AU DIAGNOSTIC DE MALADIES VASCULAIRES

(30) Priorität: 13.07.1994 DE 4425778
(43) Veröffentlichungstag der Anmeldung: 14.05.1997
(73) Patentinhaber: Institut für Diagnostikforschung GmbH an der Freien Universität Berlin, 13353 Berlin (DE)
(72) Erfinder: DINKELBORG, Ludger, D-13585 Berlin (DE); HILGER, Christoph, Stephan, D-13353 Berlin (DE); SEMMLER, Wolfhard, D-16548 Glienicke (DE); SPECK, Ulrich, D-13465 Berlin (DE); HENKLEIN, Peter, D-10365 Berlin (DE)
(74) Vertreter: Seuss, Thomas
(86) Internationale Anmeldenummer: DE9500837
(87) Internationale Veröffentlichungsnummer: WO96002568

(56) Entgegenhaltungen:
- EP-A- 0 606 683
- WO-A-91/16919
- WO-A-93/12819
- WO-A-94/22497
- J.NUCL.MED., Bd. 33, Nr. 5, 1992 Seite 845 C.KURATA ET AL 'Localisation of radioiodinated ET-1 in injured aortic tissue of cholesterol fed rabbits'
- J.NUCL.MED, Bd. 36, Nr. 5sup, 15.Juni 1995 Seite 102p L.M.DINKELBORG ET AL 'Endothelin derivatives for imaging of atherosclerosis'
- J.MED.CHEM., Bd. 35, Nr. 9, 1.Mai 1992 Seiten 1494-1508, A.M.DOHERTY 'Endothelin: a new challenge'

## Beschreibung

Die Erfindung betrifft neue Komplexverbindungen zur Diagnose von Gefäßerkrankungen, Verfahren zu deren Herstellung sowie diese Verbindungen enthaltende diagnostische Mittel.

Die Atherosklerose ist eine chronische, fortschreitende Erkrankung der Blutgefäße, die bisher klinisch erst in ihrem Spätstadium diagnostiziert werden kann. Atherosklerotische Gefäßveränderungen werden heute konventionell durch die Arteriographie dargestellt. Hierzu wird über einen Katheter ein Kontrastmittel in das jeweilige interessierende Gefäß appliziert und mittels Röntgenstrahlen solche Gefäßbereiche detektiert, die eine Verengung aufweisen. Ein Nachteil dieser Methode liegt darin, daß nur Teilbereiche des Gefäßsystems betrachtet werden können. Da die Arteriographie eine invasive Methode ist, können bei ihrer Anwendung Komplikationen wie z.B. Schmerzen, Perforationen der Arterien, Arrhythmien, Herzoder Hirninfarkte auftreten, die unter ungünstigen Umständen sogar den Tod des Patienten zur Folge haben können.

Weiterhin werden Verfahren, die auf dem Einsatz von Ultraschall beruhen, sowie die MR-Tomographie zur Diagnose der Atherosklerose eingesetzt.

Alle derzeit angewendeten Verfahren beinhalten jedoch den großen Nachteil, daß sie atherosklerotische Gefäßveränderungen durch den an diesen Stellen verminderten Blutfluß oder grobe Arterienwandveränderungen detektieren und daher nur späte Stadien der Atherogenese aufzeigen können.

Eine frühe Erkennung der Atherosklerose wäre z.B. für die Kontrolle des Therapieerfolges von Diät, Kalziumantagonisten, Lipid- und Bluthochdrucksenkern, der Kontrolle der Restenose nach Angioplastie und der Diagnose koronarer Herzerkrankungen sowie der Detektion von thrombotischen Gefäßablagerungen von großer Bedeutung.

Nicht-invasive Techniken zur Diagnose der Atherosklerose wurden beschrieben. So wurden mit Radioisotopen markierte Antikörper oder auch markierte "Low Density Lipoproteine" (LDL) vorgestellt, die an atherosklerotische Wandbereiche binden (Lees et al. 1993, J. Nucl. Med. 24, 154-156; Kaliman et al. 1985, Circulation, 72, 300; Virgolini et al. 1991, Eur. J. Nucl. Med., 18, 944-947). Diese Methoden beinhalten jedoch entscheidende Nachteile wie z.B. die antigene Wirkung der Antikörper auf den Organismus und die lange Zeitdauer (mehrere Tage), die zur Isolierung, Reinigung und Markierung des LDL aus dem Blut des Patienten benötigt wird. Vor allem aber zeigen diese großen Moleküle eine lange Halbwertszeit im Blut, die zusammen mit einer hohen Hintergrundstrahlung im gesamten Körper, eine Lokalisation der atherosklerotischen Läsionen erschwert, wenn nicht gar unmöglich macht.

Shih et al. (1990, Proc. Natl. Acad. Sci., 87, 1436-1440) synthethisierten Teilsequenzen des LDL-Proteinanteils (apo-B-100), die zwar noch an die atherosklerotischen Plaques binden, aber sich durch eine weitaus kürzere Halbwertszeit im Blut und ein verbessertes Signal-Rausch-Verhältnis hervorheben. Aufgrund einer zu geringen Affinität zum Plaque und/oder der geringen Dichte der Bindungsstellen dieser Peptide im Plaque, konnte auch mit diesen apo-B-Peptiden keine erfolgreiche *in vivo* Diagnose der Atherosklerose etabliert werden.

Der Erfindung liegt die Aufgabe zugrunde, neue Verbindungen und Mittel zur Verfügung zu stellen, die für ein nicht-invasives Verfahren zur Diagnose, insbesondere der Diagnose der frühen, noch nicht stenotischen Gefäßerkrankungen geeignet sind.

Endotheline sind physiologisch aktive Peptide, die sowohl hormonelle als auch neuroregulatorische Funktionen im Organismus ausüben (MacCumber et al. 1989, Proc. Natl. Acad. Sci., 7285-7289; Yanagisawa et al. 1989, Trends Pharmacol. Sci., 10, 374-378; LeMonier die Gouville et al., 1989, Life Sci., 45, 1499-1513; Yanagisawa et al., 1988, Nature, 332, 411-415). Vier verschiedene Isotypen konnten bisher beim Menschen nachgewiesen werden (Inoue et al., 1989, Proc. Natl. Acad. Sci., 86, 2863-2867). Endothelin 1 ist ein Polypeptid mit der folgenden Sequenz aus 21 Aminosäuren:
Cys-Ser-Cys- Ser-Ser-Leu-Met-Asp-Lys-Glu-Cys-Val-Tyr-Phe-Cys-His-Leu-Asp-Ile-Ile-Trp
(Yanagisawa et al., 1988, Nature, 332, 411-415).

Vom Gefäßendothel wird eine inaktive Vorstufe des Endothelins, das Big Endothelin gebildet. Nach Abspaltung eines Heptadecapeptids durch das Endothelin-Converting-Enzyme (ECE) entsteht Endothelin, das an spezifische Rezeptoren der glatten Gefäßmuskulatur bindet. Dort führt es zu einer Ca⁺⁺-vermittelten Kontraktion der glatten Muskelzellen (Yanagisawa et al., Nature, 332, 411-415; Takuwa et al. 1991, Contrib. Nephrol., 90, 99-104).

Eine der frühen irreversiblen Veränderungen während der Atherogenese ist die u.a. durch Wachstumsfaktoren (z.B. PDGF) hervorgerufene Proliferation glatter Muskelzellen der Gefäßwand (Desmoulière und Gabbiani 1992, Cerebrovasc. Dis., 2, 63-71). Durch die *in vitro*-Inkubation von humanen atheromatösen Koronaraterien mit ¹²⁵I-Endothelin 1 konnte gezeigt werden, daß eine vermehrte Bindung von ¹²⁵I-Endothelin 1 im Bereich der Tunica media sowie in Bereichen der Vasa vasora auftritt (Dashwood et al., 1991, J. Cardiovasc. Pharmacol., 17, 458-462). Durch die Applikation von ¹²⁵I-Endothelin 1 in Kaninchen, deren abdominale Aorta zuvor mittels Ballonkatheter deendothelisiert wurde, konnte eine vermehrte Aufnahme von ¹²⁵I-Endothelin 1 in den deendothelisierten Bereichen der Aorta gezeigt werden, die auf eine höhere Dichte der Bindungsstellen für Endothelin 1 in diesen verletzten Gefäßregionen schließen läßt (Kurata et al. 1992, J. Nucl. Med., 33, 845). Diese Untersuchungen lassen darauf schließen, daß die proliferierenden glatten Muskelzellen auch weiterhin den Endothelin-Rezeptor exprimieren.

Endothelin 1 übt eine starke vasokonstriktorische Wirkung auf die glatte Gefäßmuskulatur aus (A. M. Doherty, 1992, Medical Chemistry, 35, 1493-1508). Daher können nur relativ niedrige Konzentrationen dem Organismus i.v. appliziert werden. Höhere Konzentrationen können nur von Teilsequenzen der Endotheline, Endothelinderivate, Endothelin-Analoga bzw. Endothelin-Antagonisten, die zwar noch an den Endothelin-Rezeptor binden, nicht aber zu einer so ausgeprägten Kontraktion der glatten Muskelzellen führen, appliziert werden.

Da Endotheline sehr schnell über die Nieren ausgeschieden werden, ist die durch die Aufnahme der Endotheline in anderen Organen oder Geweben bedingte störende Hintergrundstrahlung äußerst gering.

In der deutschen Patentanmeldung P 43 01 871.8 wurden bereits Verbindungen beschrieben, welche Endotheline, Teilsequenzen von Endothelinen, Endothelin-Derivate, Endothelin-Antagonisten oder Endothelin-Analoga enthalten, die an Endothelinrezeptoren binden.

Auf der Suche nach weiteren Verbindungen wurden Substanzen gefunden in bevorzugter Weise für die nicht invasive Diagnose von Atherosklerose eignen.

Erfindungsgemäße Verbindungen sind Asp-Gly-Gly-Cys-Gly-Cys-His-Leu-Asp-Ile-Ile-Trp, Asp-Gly-Gly-Cys-Gly-Cys-D-Trp-Leu-Asp-Ile-Ile-Trp, Asp-Gly-Gly-Cys-Gly-Cys-Phe-(D-Trp)-Leu-Asp-Ile-Ile-Trp, N-(4-carboxy-1-oxo-3-thia-but-1-yl) -Cys-Phe- (D-Trp) -Leu-Asp-Ile-Ile-Trp, N- (4-carboxy-1-oxo-3-thia-but-1-yl)-Cys-(D-Trp)-Leu-Asp-Ile-Ile-Trp, N-(4-carboxy-1-oxo-3-thia-but-1-yl)-Cys-His-Leu-Asp-Ile-Ile-Trp, N-(Mercaptoacetyl)-Gly-Gly-Asp-Phe-(D-Trp)-Leu-Asp-Ile-Ile-Trp und N-(Mercaptoacetyl)-Gly-Gly-Asp-(D-Trp)-Leu-Asp-Ile-Ile-Trp.

Besonders bevorzugte erfindungsgemäße Komplexverbindungen sind Tc-99m-Komplex von Asp-Gly-Gly-Cys-Gly-Cys-His-Leu-Asp-Ile-Ile-Trp, Tc-99m-Komplex von Asp-Gly-Gly-Cys-Gly-Cys-D-Trp-Leu-Asp-Ile-Ile-Trp, Tc-99m-Komplex von Asp-Gly-Gly-Cys-Gly-Cys-Phe-(D-Trp)-Leu-Asp-Ile-Ile-Trp, Tc-99m-Komplex von N-(4-carboxy-1-oxo-3-thia-but-1-yl)-Cys-Phe-(D-Trp)-Leu-Asp-Ile-Ile-Trp, Tc-99m-Komplex von N- (4-carboxy-1-oxo-3-thia-but-1-yl)-Cys- (D-Trp)-Leu-Asp-Ile-Ile-Trp, Tc-99m-Komplex von N-(4-carboxy-1-oxo-3-thia-but-1-yl)-Cys-His-Leu-Asp-Ile-Ile-Trp, Tc-99m-Komplex von N-(Mercaptoacetyl)-Gly-Gly-Asp-Phe-(D-Trp)-Leu-Asp-Ile-Ile-Trp und Tc-99m-Komplex von N-(Mercaptoacetyl)-Gly-Gly-Asp-(D-Trp)-Leu-Asp-Ile-Ile-Trp.

Es wurde gefunden, daß sich die erfindungsgemäßen radioaktiv markierten Metallkomplexe in atherosklerotischen Gefäßläsionen überraschend stark anreichern und somit eine für die Darstellung mit einer Szintillationskamera oder anderen geeigneten, in der Nuklearmedizin angewandten Apparaturen ausreichende Konzentration erreichen. Überraschend war auch der Befund, daß die erfindungsgemäß eingesetzten Substanzen diese Konzentration in *vivo* so rasch erreichen und die Bindung so stabil ist, daß nach Abtransport und Ausscheidung der überschüssigen an den Rezeptor bindenden Substanzen eine hohe, für die Diagnostik ausreichende Konzentration verbleibt. Weiterhin war überraschend, daß die Anreicherung ganz bevorzugt an den zu diagnostizierenden, auf unterschiedlichste Weise veränderten Arterienwandbereichen erfolgt, obwohl Endothelin selbst an allen Gefäßbezirken wirkt.

Die erfindungsgemäß eingesetzten Substanzen besitzen den weiteren Vorteil, daß sie im Gegensatz zu vielen anderen geprüften Substanzklassen und Substanzen sich nicht zusätzlich und unspezifisch in anderen Geweben und Organen anreichern, was für eine Eignung als Diagnostikum entscheidend ist.

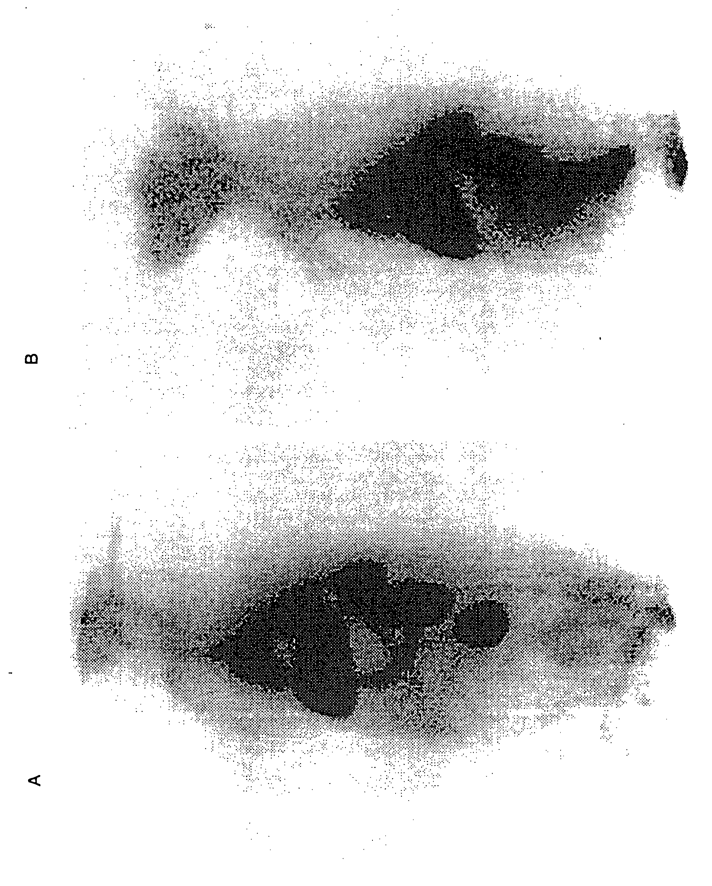

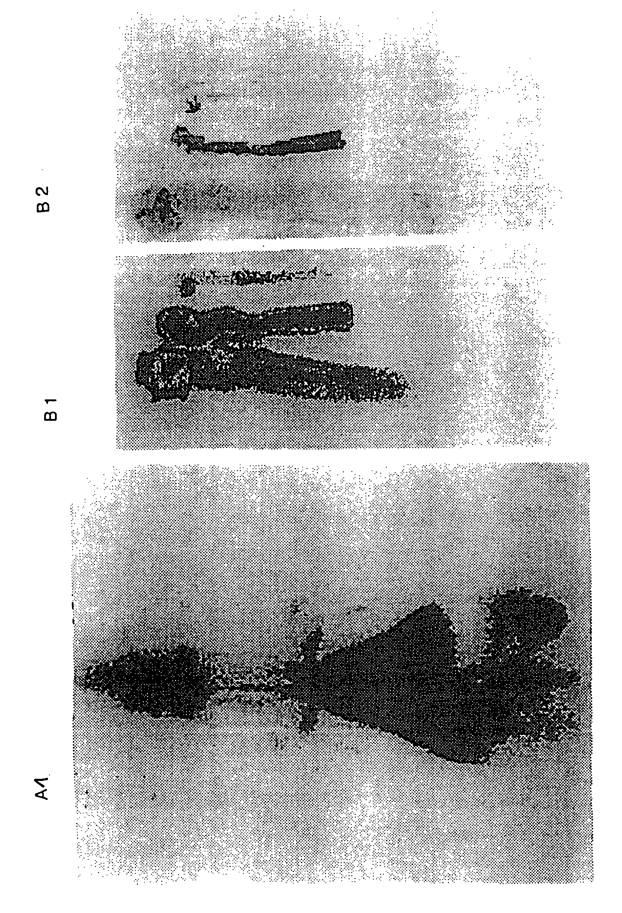

Die Fig. 1 und 2 zeigen die Anreicherung der erfindungsgemäßen Verbindungen in vivo.
Fig. 1 stellt ein linkslaterales Szintigramm eines WHHL-Kaninchens (A) bzw. eines Neuseeländer Kontrollkaninchens (B) 0,5 h nach i.v. Applikation von 74 MBq Tc-99m-Komplex von Asp-Gly-Gly-Cys-Gly-Cys-Phe-(D-Trp)-Leu-Asp-Ile-Ile-Trp (vgl. Beispiel 3 c) dar. Aktivitätsanreicherungen im Bereich des Aortenbogens, der linken a. carotis sowie in Bereichen der abdominalen Aorta sind in WHHL-Kaninchen (A) im Gegensatz zu Kontrollkaninchen (B) in vivo sichtbar.
Fig. 2 zeigt ein anteriores Szintigramm eines Neuseeländer Kaninchens, deren rechte a carotis vier Wochen zuvor mit Hilfe eines Fogarty-Katheders denudiert wurde (A), 0,5 h nach i.v. Applikation von 74 MBq Tc-99m-Komplex von Asp-Gly-Gly-Cys-Gly-Cys-(D-Trp)-Leu-Asp-Ile-Ile-Trp (vgl. Beispiel 2 c), sowie ein Autoradiogramm (B1) und Sudan-III-Färbung (B2) der 5 h nach i.v. Applikation herauspräparierten thorakalen Aorta der denudierten und der unbehandelten a. carotis. Die Reihenfolge der herauspräparierten Gefäße bei B1 und B2 jeweils von links nach rechts: thorokale Aorta, rechte denudierte a carotis und linke unbehandelte a. carotis. Die stark atherosklerotische rechte a carotis konnte in vivo detektiert werden (A). Die in vitro Untersuchungen (B1 und B2) bestätigen die gute Korrelation zwischen hoher und selektiver Akkumulation der erfindungsgemäßen Verbindungen in der denudierten rechten a. carotis im Vergleich zur unbehandelten linken a. carotis.

Die erfindungsgemäßen Verbindungen und die aus ihnen bereiteten Lösungen erfüllen die genannten Anforderungen in überraschend hohem Maße. Sie besitzen sowohl eine höhere Anreicherung in pathologischen Gefäßbereichen als auch eine durch die günstigere Pharmakokinetik bedingte bessere Kontrasteigenschaft, als die bisher für die Erfassung von Gefäßerkrankungen beschriebenen Diagnostika. Die praktische Anwendung der neuen erfindungsgemäßen Substanzen wird auch durch deren hohe chemische Stabilität erleichtert.

In der Tabelle 1 ist die Anreicherung (cpm/mm²) der erfindungsgemäßen Verbindungen im Plaque sowie in nicht verplaquten Bereichen der Aorta sowie der Anreicherungsfaktor (Quotient aus Anreicherung im Plaque und Anreicherung in nicht verplaquten Aortengewebe) dargestellt. 74 MBq (2 mCi) der jeweiligen erfindungsgemäßen Verbindung wurden einem WHHL-Kaninchen über eine Ohrvene appliziert. 5 h p.i. wurden die Kaninchen getötet und sowohl eine quantitative Autoradiographie als auch eine Sudan-III-Färbung der entnommenen Aorta durchgeführt. Die so berechnete Akkumulation der jeweiligen erfindungsgemäßen Verbindungen in den verschiedenen Geweben ist in Tabelle 1 dargestellt.

**Tabelle 1**

| | Anreicherung (cpm/mm²) | | Anreicherungsfaktor |
|---|---|---|---|
| Nummer des Beispiels | Plaque | nicht Plaque | Plaque / nicht Plaque |
| 1 c | 501 | 89 | 6 |
| 2 c | 3090 | 431 | 7 |
| 3 c | 3930 | 368 | 11 |
| 4 c | 3912 | 274 | 14 |
| 5 c | 833 | 98 | 9 |
| 6 c | 353 | 76 | 5 |
| 7 c | 619 | 48 | 13 |
| 8 c | 513 | 52 | 10 |

Die Komplexbildnerreste, welche ebenfalls in den erfindungsgemäßen Komplexen enthalten sind, müssen in der Lage sein, die jeweiligen Metallionen in Form von koordinativen und/oder kovalenten Bindungen zu binden.

Je nach Art des zu bindenden Metallions, in Abhängigkeit von deren Ladung, Oxidationszahl und Atom- bzw. Ionenradius muß der Komplexbildnerrest derart gewählt werden, daß eine effektive und für die erfindungsgemäße Verwendung der erfindungsgemäßen Komplexe ausreichende Bindung erzeugt wird.

Die Herstellung der erfindungsgemäßen Komplexe mit Metallionen erfolgt nach den dem Fachmann bekannten Verfahren, in dem in an sich bekannter Weise ein radioaktives Metallion in Form seines Permetallates, in Anwesenheit eines Reduktionsmittels und gegebenenfalls eines Hilfsliganden mit einer Verbindung der allgemeinen Formel (I) umgesetzt wird.

Metallionen sind ^{99m}Tc oder Re in Form von Pertechnat oder Perrhenat.

Die Reaktion wird bevorzugt in wäßrigem Medium bei Raumtemperatur durchgeführt. die Abspaltung der SH-Schutzgruppen erfolgt in situ oder nach den dem Fachmann bekannten Literaturmethoden, zum Beispiel durch basische Hydrolyse, reduktive Spaltung, etc. (siehe zum Beispiel "Protective Groups in Organic Synthesis", T. W. Greene, John Wiley and Sons 1981).

Die Herstellung der erfindungsgemäßen Komplexe mit Metallionen erfolgt ferner auch dadurch, daß man in an sich bekannter Weise ein geeignetes Salz oder Oxid eines geeignteten radioaktiven Kations mit einer Verbindung umsetzt.

Die Herstellung cysteinreicher Aminosäuresequenzen von Metallothioninen (siehe Patentanmeldung WO 91/17173) erfolgt in analoger Weise.

Die Herstellung der Peptide mit dem C-terminalen Tripeptid Ile-Ile-Trp erfolgte nach literaturbekannten Standardverfahren (Barany and Merrifield, The Peptides: Analysis, Synthesis, Biology Academic Press, New York, 1980; Stewart and Young, Solid-Phase Peptide Synthesis, 2nd ed., Pierce Chemical Co., Rockford, IL, 1984; Atherton and Sheppard, Solid-Phase Peptide Synthesis, IRL Press, Oxford, England, 1989).

Die vorliegende Erfindung stellt ferner diagnostische Mittel zur Verfügung, welche durch den Gehalt eines Komplexes der Verbindung der Erfindung mit Tc und Re gekennzeichnet sind. Durch die geeignete Wahl des Metallions sind die Mittel für unterschiedliche diagnostische Verfahren geeignet.

Ist das erfindungsgemäße Mittel zur Anwendung in der Radiodiagnostik bestimmt, so muß das Zentralion des Komplexsalzes radioaktiv sein. Bevorzugte Isotope sind ^{99m}Tc, ¹⁸⁶Re.

Gegenstand der vorliegenden Erfindung sind ferner Verfahren zur Herstellung der erfindungsgemäßen diagnostischen Mittel.

Die Herstellung der erfindungsgemäßen radiopharmazeutischen Mittel erfolgt nach an sich bekannter Weise, indem man die erfindungsgemäßen Komplexbildner und deren Konjugate - gegebenenfalls unter Zugabe der in der Galenik üblichen Zusätze - in wäßrigem Medium löst oder suspendiert und anschließend die Lösung oder Suspension gegebenenfalls lyophilisiert oder sterilisiert. Geeignete Zusätze sind beispielsweise physiologisch unbedenkliche Puffer (wie zum Beispiel Tromethamin), Zusätze von Hilfsliganden (wie zum Beispiel Natriumcitrat oder Natriumtartrat), Reduktionsmittel (wie zum Beispiel Zinn(II)-chlorid) oder - falls erforderlich - Elektrolyte wie zum Beispiel Natriumchlorid oder, falls erforderlich,(einen) in der Galenik üblichen Hilfsstoff(e) (zum Beispiel Lactose, Methylcellulose, Mannit) und/oder Tensid(en) (zum Beispiel Lecithine, Tween®, Myrj®). Die verwendeten Zusätze müssen in ihrer Zusammensetzung eine Herstellung der erfindungsgemäßen Verbindungen erlauben.

Bei der nuklearmedizinischen *in vivo*-Anwendung werden die erfindungsgemäßen Mittel in Mengen von 1x10⁻⁵ bis 5x10⁴ nmol/kg Körpergewicht, vorzugsweise in Mengen zwischen 1x10⁻³ bis 5x10² nmol/kg Körpergewicht dosiert. Ausgehend von einem mittleren Körpergewicht von 70 kg beträgt die Radioaktivitätsmenge für diagnostische Anwendungen zwischen 0,05 und 50 mCi, vorzugsweise 5 bis 30 mCi pro Applikation. Die Applikation erfolgt normalerweise durch intravenöse, intraarterielle oder peritoneale Injektion von 0,1 bis 5 ml einer Lösung der erfindungsgemäßen Mittel. Bevorzugt ist die intravenöse Applikation. Details ihrer Anwndung und Dosierung werden zum Beispiel in "Radiotracers for Medical Applications", CRC-Press, Boca Raton, Florida, beschrieben.

Die erfindungsgemäßen radiopharmazeutischen Mittel erfüllen die vielfältigen Voraussetzungen für die Eignung als Radiopharmaka für die Radiodiagnostik und die Radiotherapie. So sind sie hervorragend dazu geeignet, sich nach i.v. Applikation in Zielgeweben anzureichern und ermöglichen so eine nicht-invasive Diagnose entsprechender Gewebe. Die Wasserlöslichkeit der erfindungsgemäßen radiopharmazeutischen Mittel wird- falls erfoderlichdurch die in der Galenik üblichen Hilfsstoffe wie oben beschrieben gewährleistet.

Weiterhin weisen die erfindungsgemäßen radiopharmazeutischen Mittel nicht nur eine hohe Stabilität *in vitro* auf, sondern auch eine überraschend hohe Stabilität in *vivo,* so daß eine Freigabe oder ein Austausch des im Komplex gebundenen Radionuklids nicht oder klinisch nicht relevant erfolgt.

Die Herstellung der erfindungsgemäßen pharmazeutischen Mittel erfolgt ferner in an sich bekannter Weise, indem man die erfindungsgemäßen Komplexbildner unter Zusatz eines Reduktionsmittels, vorzugsweise Zinn-(II)-salzen wie -chlorid oder -tartrat und -gegebenenfall unter Zugabe der in der Galenik üblichen Zusätze - in wäßrigem Medium löst und anschließend sterilfiltriert. Geeignete Zusätze sind beispielsweise physiologisch unbedenkliche Puffer (z.B. Tromethamin), geringe Zusätze von Elektrolyten (z.B. Natriumchlorid), Stabilisatoren (z.B. Gluconat, Phosphate oder Phosphonate). Das erfindungs-gemäße pharmazeutische Mittel liegt in Form einer Lösung oder in lyophilisierter Form vor und wird kurz vor der Applikation beispielsweise mit einer Lösung Tc-99m-Pertechnetat, eluiert aus kommerziell erhältlichen Generatoren, oder einer Perrhenatlösung versetzt.

Zur Herstellung der Radiopharmaka wird ein erfindungsgemäßes Cold Kit zur Verfügung gestellt. Dieses Cold Kit beinhaltet eine erfindungsgemäße Verbindung der allgemeinen Formel (I), ein Reduktionsmittel und gegebenenfalls einen oder mehrere Hilfsliganden in Lösung, in trockenem Zustand oder in lyophilisierter Form. Das Cold Kit umfaßt ferner eine Gebrauchsanweisung mit einer Reaktionsvorschrift zur Umsetzung der erfindungsgemäßen Verbindungen mit einem Permetallat eines radioaktiven Metallions unter Bildung eines erfindungsgemäßen Komplexes einer Verbindung der allgemeinen Formel (I) mit dem Metallion.

Die Verwendung des Cold Kits, das aus einem verschließbaren Gefäß besteht, welches eine vorbestimmte Menge von eine Verbindung der Efindung, der in der Lage ist, Metallatome zu binden, und außerdem eine ausreichende Menge eines Reduktionsmittsl enthält, um die Verbindung mit ^{99m}Tc zu markieren und zur Herstellung einer radiopharmazeutischen Zubereitung dient, ist ebenfalls ein Gegenstand der vorliegenden Erfindung.

Gegenstand der vorliegenden Erfindung ist ferner ein Verfahren zur bildlichen Darstellung pathologischer Gefäßveränderungen, welches sich dadurch auszeichnet, daß als Kontrastmittel ein Komplex der Verbindung der Erfindung mit Metallionen Tc oder Re eingesetzt wird.

In einer Methode zur Durchführung einer radiodiagnostischen Untersuchung wird die radiopharmazeutische Zusammensetzung in einer Menge von 0,1 bis 30mCi, bevorzugt von 0,5 bis 10 mCi pro 70 kg Körpergewicht einem Patienten verabreicht und die vom Patienten abgegebene Strahlung aufgezeichnet.

Die nachfolgenden Beispiele erläutern die Erfindung:

### Beispiel 1

a) Synthese von Asp-Gly-Gly-Cys-Gly-Cys-His-Leu-Asp-Ile-Ile-Trp.
Asp-Gly-Gly-Cys-Gly-Cys-His-Leu-Asp-Ile-Ile-Trp wurde in Analogie zu Barany und Marrifield, The Peptides: Analysis, Biology, Academic Press, New York, 1980; Stewart und Young, Solid-'Phase Peptide Synthesis, 2nd ed., Pierce Chemical Co., Rockford, IL, 1984 hergestellt.

| | | |
|---|---|---|
| Molekulargewicht | ber. | 1288,4 |
| | best. | 1288 (FAB-MS) |

b) ^{99m}Tc-Komplex von Asp-Gly-Gly-Cys-Gly-Cys-His-Leu-Asp-Ile-Ile-Trp.
0,5 mg des Asp-Gly-Gly-Cys-Gly-Cys-His-Leu-Asp-Ile-Ile-Trp in 300 µl Phosphatpuffer (Na₂HPO₄, 0,5 mol/l pH 8,5) werden mit 50 µl einer 0,15 molaren Trinatriumcitratdihydrat-Lösung und 2,5 µl einer 0,2 molaren Zinn(II)-chlorid-Dihydrat-Lösung versetzt. Das Reaktionsgemisch wird mit einer Pertechnetatlösung (0,4 bis 0,9 mCi) aus einem Mo-99/Tc-99m-Generator versetzt, 10 min bei Raumtemperatur inkubiert. Die Analytik der Markierung erfolgt über HPLC.
c) In vivo und in vitro Anreicherung des ^{99m}Tc-Komplex von Asp-Gly-Gly-Cys-Gly-Cys-His-Leu-Asp-Ile-Ile-Trp in WHHL-Kaninchen.
2 mCi (1 ml) des analog zu b) markierten Asp-Gly-Gly-Cys-Gly-Cys-His-Leu-Asp-Ile-Ile-Trp wurde einem narkotisierten WHHL-Kaninchen (Rompun/Ketavet 1:2) über eine Ohrvene appliziert. WHHL-Kaninchen weisen aufgrund eines fehlenden oder defekten LDL-Rezeptors hohe LDL-Spiegel im Blut auf und bilden daher spontan atherosklerotische Gefäßveränderungen aus. Während des Versuchszeitraumes von 5 h nach Applikation wurden statische Aufnahmen verschiedener Belichtungszeiten und aus verschiedenen Positionen mit einer Gammakamera (Elcint SP4 HR) angefertigt. 5h nach Applikation wurde das Kaninchen getötet und sowohl eine Autoradiographie der Aorta als auch eine Sudan III-Färbung durchgeführt. Die atherosklerotischen Plagues im Bereich des Aortabogens von WHHL-Kaninchen konnte 10 min p.i. in vivo dargestellt werden. Die anschließend durchgeführte Autoradiographie ergab eine Markierung der gesamten Aortenwand sowie der atherosklerotischen Plaques. Der Anreichungsfaktor zwischen normalen und atherosklerotischen Wandbereichen betrug je nach Ausbildung der Plaques (Sudan III-Färbung) zwischen 6 und 9. Die Ergebisse sind in Tabelle 1 zusammengefaßt.

### Beispiel 2

a) Synthese von Asp-Gly-Gly-Cys-Gly-Cys-D-Trp-Leu-Asp-Ile-Ile-Trp.
Asp-Gly-Gly-Cys-Gly-Cys-D-Trp-Leu-Asp-Ile-Ile-Trp wurde in Analogie zu Barany und Marrifield, The Peptides: Analysis, Biology, Academic Press, New York, 1980; Stewart und Young, Solid-Phase Peptide Synthesis, 2nd ed., Pierce Chemical Co., Rockford, IL, 1984 hergestellt.

| | | |
|---|---|---|
| Molekulargewicht | ber. | 1337,5 |
| | best. | 1337 (FAB-MS) |

b) ^{99m}Tc-Komplex von Asp-Gly-Gly-Cys-Gly-Cys-D-Trp-Leu-Asp-Ile-Ile-Trp.
0,5 mg des Asp-Gly-Gly-Cys-Gly-Cys-D-Trp-Leu-Asp-Ile-Ile-Trp in 300 µl Phosphatpuffer (Na₂HPO₄, 0,5 mol/l, pH 8,5) werden mit 50 µl einer 0,15 molaren Trinatriumcitratdihydrat-Lösung und 2,5 µl einer 0,2 molaren Zinn(II)-chlorid-Dihydrat-Lösung versetzt. Das Reaktionsgemisch wird mit einer Pertechnetatlösung (0,4 bis 0,9 mCi) aus einem Mo-99/Tc-99m-Generator versetzt, 10 min bei Raumtemperatur inkubiert. Die Analytik der Markierung erfolgt über HPLC.
c) In vivo und in vitro Anreicherung des ^{99m}Tc-Komplex von Asp-Gly-Gly-Cys-Gly-Cys-D-Trp-Leu-Asp-Ile-Ile-Trp in WHHL-Kaninchen.
Die in vivo und in vitro Anreicherung des ^{99m}Tc-Komplex von Asp-Gly-Gly-Cys-Gly-Cys-D-Trp-Leu-Asp-Ile-Ile-Trp in WHHL-Kaninchen wurde wie in Beispiel 1 c) beschrieben durchgeführt. Die Ergebnisse sind in Tabelle 1 zusammengefaßt.

### Beispiel 3

a) Synthese von Asp-Gly-Gly-Cys-Gly-Cys-Phe-(D-Trp)-Leu-Asp-Ile-Ile-Trp.
Asp-Gly-Gly-Cys-Gly-Cys-Phe-(D-Trp)-Leu-Asp-Ile-Ile-Trp wurde in Analogie zu Barany und Marrifield, The Peptides: Analysis, Biology, Academic Press, New York, 1980; Stewart und Young, Solid-Phase Peptide Synthesis, 2nd ed., Pierce Chemical Co., Rockford, IL, 1984 hergestellt.

| | | |
|---|---|---|
| Molekulargewicht | ber. | 1484,68 |
| | best. | 1484 (FAB-MS) |

b) ^{99m}Tc-Komplex von Asp-Gly-Gly-Cys-Gly-Cys-Phe-(D-Trp)-Leu-Asp-Ile-Ile-Trp
0,5 mg des Asp-Gly-Gly-Cys-Gly-Cys-Phe-(D-Trp)-Leu-Asp-Ile-Ile-Trp in 300 µl Phosphatpuffer (Na₂HPO₄, 0,5 mol/l, pH 8,5) werden mit 50 µl einer 0.15 molaren Trinatriumcitratdihydrat-Lösung und 2,5 µl einer 0,2 molaren Zinn(II)chlorid-Dihydrat-Lösung versetzt. Das Reaktionsgemisch wird mit einer Pertechnetatlösung (0,4 bis 0,9 mCi) aus einem Mo-99/Tc-99m-Generator versetzt, 10 min bei Raumtemperatur inkubiert. Die Analytik der Markierung erfolgt über HPLC.
c) In vivo und in vitro Anreicherung des Tc-99m-Komplex von Asp-Gly-Gly-Cys-Gly-Cys-Phe-(D-Trp)-Leu-Asp-Ile-Ile-Trp in WHHL-Kaninchen.
Die in vivo und in vitro Anreicherung des Tc-99m-Komplex von Asp-Gly-Gly-Cys-Gly-Cys-Phe-(D-Trp)-Leu-Asp-Ile-Ile-Trp in WHHL-Kaninchen wurde wie in Beispiel 1 c) beschrieben durchgeführt. Die Ergebnisse sind in Tabelle 1 zusammengefaßt.

### Beispiel 4

a) Synthese von N-(4-carboxy-1-oxo-3-thia-but-1-yl)-Cys-Phe-(D-Trp)-Leu-Asp-Ile-Ile-Trp
109,5 mg (0,1 mmol) NH₂-Cys-Phe-(D-Trp)-Leu-Asp-Ile-Ile-Trp (hergestellt in Analogie zu: E. Atherton, R.C. Sheppard, Solid phase peptide synthesis, Oxford university press, Oxford, New York, Tokyo) werden unter Argonatmosphäre in 3 ml wasserfreiem Dimethylformamid unter Zugabe von 30,36 mg (0,3 mmol) Triethylamin in Lösung gebracht. Bei 0°C versetzt man mit 13,21 mg (0,1 mmol) Thiodiglycolsäureanhydrid. Anschließend wird 2h bei 0°C und abschließend über Nacht bei Raumtemperatur nachgerührt. Das Rohpeptid wird durch Zugabe von 10 ml wasserfreiem Diethylether ausgefällt und durch Chromatographie an Kieselgel RP-18 (Eluens: Wasser/0,1% Trifluoressigsäure, Acetonitril/0,1% Trifluoressigsäure, linearer Gradient) gereinigt. Nach Lyophilisieren erhält man ein weißes Pulver.
Ausbeute: 48,5 mg (39,5 % d. Th.), weißes Pulver

| | | |
|---|---|---|
| Molekulargewicht | ber. | 1227,4438 |
| | best. | 1227 (FAB-MS) |

b) Tc-99m-Komplex von N-(4-Hydroxycarboxy-1-oxo-3-thiabut-1-yl)-Cys-Phe-(D-Trp)-Leu-Asp-Ile-Ile-Trp
1 mg des unter Beispiel 4 a) beschriebenen Peptidkonjugates HOOC-CH₂-S-CH₂-CONH-Cys-Phe-(D-Trp)-Leu-Asp-Ile-Ile-Trp wird in 500 µl Dinatriumhydrogen-phosphatpuffer (0,01 mol/l, pH 8,5) gelöst und mit 50 µl einer 0,15 molaren Trinatriumcitratdihydratlösung versetzt. Nach Zugabe von Pertechnetatlösung (0,4-0,9 mCi) aus einem Mo-99/Tc-99m-Generator gibt man 5 µl einer 0,2 molaren Zinn-(II)-chloriddihydratlösung hinzu und inkubiert 10 min bei Raumtemperatur. Die Markierungsausbeute wird mittels analytischer HPLC bestimmt und ist größer 95%.
c) In vivo und in vitro Anreicherung des Tc-99m-Komplex von N-(4-carboxy-1-oxo-3-thia-but-1-yl)-Cys-Phe-(D-Trp)-Leu-Asp-Ile-Ile-Trp in WHHL-Kaninchen.
Die in vivo und in vitro Anreicherung des Tc-99m-Komplex von N-(4-carboxy-1-oxo-3-thia-but-1-yl)-Cys-Phe-(D-Trp)-Leu-Asp-Ile-Ile-Trp in WHHL-Kaninchen wurde wie in Beispiel 1 c) beschrieben durchgeführt. Die Ergebnisse sind in Tabelle 1 zusammengefaßt.

### Beispiel 5

a) Synthese von N-(4-carboxy-1-oxo-3-thia-but-1-yl)-Cys-(D-Trp)-Leu-Asp-Ile-Ile-Trp
94,8 mg (0,1 mmol) NH₂-Cys-(D-Trp)-Leu-Asp-Ile-Ile-Trp (hergestellt in Analogie zu: E. Atherton, R.C. Sheppard, Solid phase peptide synthesis, Oxford university press, Oxford, New York, Tokyo) werden unter Argonatmosphäre in 3 ml wasserfreiem Dimethylformamid unter Zugabe von 30,36 mg (0,3 mmol) Triethylamin in Lösung gebracht. Bei 0°C versetzt man mit 13,21 mg (0,1 mmol) Thiodiglycolsäureanhydrid. Anschließend wird 2h bei 0°C und abschließend über Nacht bei Raumtemperatur nachgerührt. Das Rohpeptid wird durch Zugabe von 10 ml wasserfreiem Diethylether ausgefällt und durch Chromatographie an Kieselgel RP-18 (Eluens: Wasser/0,1% Trifluoressigsäure, Acetonitril/0,1% Trifluoressigsäure, linearer Gradient) gereinigt. Nach Lyophilisieren erhält man ein weißes Pulver.
Ausbeute: 39,3 mg (36,4 % d. Th.), weißes Pulver

| | | |
|---|---|---|
| Molekulargewicht | ber. | 1080,2738 |
| | best. | 1080 (FAB-MS) |

b) Tc-99m-Komplex von N-(4-carboxy-1-oxo-3-thia-but-1-yl)-Cys-(D-Trp)-Leu-Asp-Ile-Ile-Trp
1 mg des unter Beispiel 5 a) beschriebenen Peptidkonjugates HOOC-CH₂-S-CH₂-CONH-Cys-(D-Trp)-Leu-Asp-Ile-Ile-Trp wird in 500 µl Dinatriumhydrogenphos-phatpuffer (0,01 mol/l, pH 8,5) gelöst und mit 50 µl einer 0,15 molaren Trinatriumcitratdihydratlösung versetzt. Nach Zugabe von Pertechnetatlösung (0,4-0,9 mCi) aus einem Mo-99/Tc-99m-Generator gibt man 5 µl einer 0,2 molaren Zinn-(II)-chloriddihydratlösung hinzu und inkubiert 10 min bei Raumtemperatur. Die Markierungsausbeute wird mittels analytischer HPLC bestimmt und ist größer 95%.
c) In vivo und in vitro Anreicherung des Tc-99m-Komplex von N-(4-carboxy-1-oxo-3-thia-but-1-yl)-Cys-(D-Trp)-Leu-Asp-Ile-Ile-Trp in WHHL-Kaninchen.
Die in vivo und in vitro Anreicherung des Tc-99m-Komplex von N-(4-carboxy-1-oxo-3-thia-but-1-yl)-Cys- (D-Trp)-Leu-Asp-Ile-Ile-Trp in WHHL-Kaninchen wurde wie in Beispiel 1 c) beschrieben durchgeführt. Die Ergebnisse sind in Tabelle 1 zusammengefaßt.

### Beispiel 6

a) Synthese von N-(4-carboxy-1-oxo-3-thia-but-1-yl)-Cys-His-Leu-Asp-Ile-Ile-Trp
89,9 mg (0,1 mmol) NH₂-Cys-His-Leu-Asp-Ile-Ile-Trp (hergestellt in Analogie zu: E. Atherton, R.C. Sheppard, Solid phase peptide synthesis, Oxford university press, Oxford, New York, Tokyo) werden unter Argonatmosphäre in 3 ml wasserfreiem Dimethylformamid unter Zugabe von 30,36 mg (0,3 mmol) Triethylamin in Lösung gebracht. Bei 0°C versetzt man mit 13,21 mg (0,1 mmol) Thiodiglycolsäureanhydrid. Anschließend wird 2h bei 0°C und abschließend über Nacht bei Raumtemperatur nachgerührt. Das Rohpeptid wird durch Zugabe von 10 ml wasserfreiem Diethylether ausgefällt und durch Chromatographie an Kieselgel RP-18 (Eluens: Wasser/0,1% Trifluoressigsäure, Acetonitril/0,1% Trifluoressigsäure, linearer Gradient) gereinigt. Nach Lyophilisieren erhält man ein weißes Pulver.
Ausbeute: 26,9 mg (26,1 % d. Th.), weißes Pulver

| | | |
|---|---|---|
| Molekulargewicht | ber. | 1031,2038 |
| | best. | 1031 (FAB-MS) |

b) Tc-99m-Komplex von N-(4-carboxy-1-oxo-3-thia-but-1-yl)-Cys-His-Leu-Asp-Ile-Ile-Trp
1 mg des unter Beispiel 6 a) beschriebenen Peptidkonjugates HOOC-CH₂-S-CH₂-CONH-Cys-His-Leu-Asp-Ile-Ile-Trp wird in 500 µl Dinatriumhydrogenphosphat-puffer (0,01 mol/l, pH 8,5) gelöst und mit 50 µl einer 0,15 molaren Trinatrium-citratdihydratlösung versetzt. Nach Zugabe von Pertechnetatlösung (0,4-0,9 mCi) aus einem Mo-99/Tc-99m-Generator gibt man 5 µl einer 0,2 molaren Zinn-(II)-chloriddihydratlösung hinzu und inkubiert 10 min bei Raumtemperatur. Die Markierungsausbeute wird mittels analytischer HPLC bestimmt und ist größer 95%.
c) In vivo und in vitro Anreicherung des Tc-99m-Komplex von N-(4-Hydroxycarboxy-1-oxo-3-thia-but-1-yl)-Cys-His-Leu-Asp-Ile-Ile-Trp in WHHL-Kaninchen.
Die in vivo und in vitro Anreicherung des Tc-99m-Komplex von N-(4-Hydroxycarboxy-l-oxo-3-thia-but-1-yl)-Cys-His-Leu-Asp-Ile-Ile-Trp in WHHL-Kaninchen wurde wie in Beispiel 1 c) beschrieben durchgeführt. Die Ergebnisse sind in Tabelle 1 zusammengefaßt.

### Beispiel 7

a) Synthese von N-(Mercaptoacetyl)-Gly-Gly-Asp-Phe-(D-Trp)-Leu-Asp-Ile-Ile-Trp
122,1 mg (0,1 mmol) NH₂-Gly-Gly-Asp-Phe-(D-Trp)-Leu-Asp-Ile-Ile-Trp (hergestellt in Analogie zu: E. Atherton, R.C. Sheppard, Solid phase peptide synthesis, Oxford university press, Oxford, New York, Tokyo) werden unter Argonatmosphäre in 3 ml wasserfreiem Dimethylformamid unter Zugabe von 40,48 mg (0,4 mmol) Triethylamin in Lösung gebracht. Bei 0°C versetzt man mit einer Lösung von S-Trityl-mercaptoessigsäure-N-hydroxysuccinimidester in 3 ml wasserfreiem Dimethylformamid [Herstellung: 36,79 mg (0.11 mmol) S-Trityl-mercaptoessigsäure werden unter Zugabe von 12,66 mg (0,11 mmol) N-Hydroxysuccinimid in 2 ml wasserfreiem Dimethylformamid in Lösung gebracht. Man versetzt mit einer Lösung von 22,70 mg (0,11 mmol) Dicyclohexylcarbodiimid in 1ml wasserfreiem Dimethylformamid und inkubiert 30 min. Nach Filtrieren kann die so in situ präparierte S-Trityl-mercaptoessigsäure-N-hydroxysuccinimidester-Lösung zur Kopplung verwendet werden.]. Anschließend wird 2h bei 0°C und abschließend über Nacht bei Raumtemperatur nachgerührt. Das Rohpeptid wird durch Zugabe von 20 ml wasserfreiem Diethylether ausgefällt und für 10 min in 5 ml Trifluoressigsäure/5% Wasser/22 mg Tributylsilan unter Argonatmosphäre gerührt. Das schutzgruppenfreie Rohpeptid wird durch Fällung mit 25 ml wasserfreiem Diethylether gewonnen und durch Chromatographie an Kieselgel RP-18 (Eluens: Wasser/0,1% Trifluoressigsäure, Acetonitril/0,1% Trifluoressigsäure, linearer Gradient) gereinigt. Nach Lyophilisieren erhält man ein weißes Pulver.
Ausbeute: 56,3 mg (43,4 % d. Th.), weißes Pulver

| | | |
|---|---|---|
| Molekulargewicht | ber. | 1296,4551 |
| | best. | 1296 (FAB-MS) |

b) Tc-99m-Komplex von N-(Mercaptoacetyl)-Gly-Gly-Asp-Phe-(D-Trp)-Leu-Asp-Ile-Ile-Trp
1 mg des unter Beispiel 7 a) beschriebenen Peptidkonjugates HS-CH₂-CONH-Gly-Gly-Asp-Phe-(D-Trp)-Leu-Asp-Ile-Ile-Trp wird in 500 µl Dinatriumhydrogen-phosphatpuffer (0,01 mol/l, pH 8,5) gelöst und mit 50 µl einer 0,15 molaren Trinatriumcitratdihydratlösung versetzt. Nach Zugabe von Pertechnetatlösung (0,4-0,9 mCi) aus einem Mo-99/Tc-99m-Generator gibt man 5 µl einer 0,2 molaren Zinn-(II)-chloriddihydratlösung hinzu und inkubiert 10 min bei Raumtemperatur. Die Markierungsausbeute wird mittels analytischer HPLC bestimmt und ist größer 98%.
c) In vivo und in vitro Anreicherung des Tc-99m-Komplex von N-(Mercaptoacetyl)-Gly-Gly-Asp-Phe-(D-Trp)-Leu-Asp-Ile-Ile-Trp in WHHL-Kaninchen.
Die in vivo und in vitro Anreicherung des Tc-99m-Komplex von N-(Mercaptoacetyl)-Gly-Gly-Asp-Phe-(D-Trp)-Leu-Asp-Ile-Ile-Trp in WHHL-Kaninchen wurde wie in Beispiel 1 c) beschrieben durchgeführt. Die Ergebnisse sind in Tabelle 1 zusammengefaßt.

### Beispiel 8

a) Synthese von N-(Mercaptoacetyl)-Gly-Gly-Asp-(D-Trp)-Leu-Asp-Ile-Ile-Trp
107,4 mg (0,1 mmol) NH₂-Gly-Gly-Asp-(D-Trp)-Leu-Asp-Ile-Ile-Trp (hergestellt in Analogie zu: E. Atherton, R.C. Sheppard, Solid phase peptide synthesis, Oxford university press, Oxford, New York, Tokyo) werden unter Argonatmosphäre in 3 ml wasserfreiem Dimethylformamid unter Zugabe von 40,48 mg (0,4 mmol) Triethylamin in Lösung gebracht. Bei 0°C versetzt man mit einer Lösung von S-Trityl-mercaptoessigsäure-N-hydroxysuccinimidester in 3 ml wasserfreiem Dimethylformamid [Herstellung: 36,79 mg (0,11 mmol) S-Trityl-mercaptoessigsäure werden unter Zugabe von 12,66 mg (0,11 mmol) N-Hydroxysuccinimid in 2 ml wasserfreiem Dimethylformamid in Lösung gebracht. Man versetzt mit einer Lösung von 22,70 mg (0,11 mmol) Dicyclohexylcarbodiimid in 1ml wasserfreiem Dimethylformamid und inkubiert 30 min. Nach Filtrieren kann die so in situ präparierte S-Trityl-mercaptoessigsäure-N-hydroxysuccinimidester-Lösung zur Kopplung verwendet werden.]. Anschließend wird 2h bei 0°C und abschließend über Nacht bei Raumtemperatur nachgerührt. Das Rohpeptid wird durch Zugabe von 20 ml wasserfreiem Diethylether ausgefällt und für 10 min in 5 ml Trifluoressigsäure/5% Wasser/22 mg Tributylsilan unter Argonatmosphäre gerührt. Das schutzgruppenfreie Rohpeptid wird durch Fällung mit 25 ml wasserfreiem Diethylether gewonnen und durch Chromatographie an Kieselgel RP-18 (Eluens: Wasser/0,1% Trifluoressigsäure, Acetonitril/0,1% Trifluoressigsäure, linearer Gradient) gereinigt. Nach Lyophilisieren erhält man ein weißes Pulver.
Ausbeute: 46,3 mg (40,3 % d. Th.), weißes Pulver

| | | |
|---|---|---|
| Molekulargewicht | ber. | 1049,2851 |
| | best. | 1049 (FAB-MS) |

b) Tc-99m-Komplex von N-(Mercaptoacetyl)-Gly-Gly-Asp-(D-Trp)-Leu-Asp-Ile-Ile-Trp
1 mg des unter Beispiel 8 a) beschriebenen Peptidkonjugates HS-CH₂-CONH-Gly-Gly-Asp- (D-Trp)-Leu-Asp-Ile-Ile-Trp wird in 500 µl Dinatriumhydrogenphos-phatpuffer (0,01 mol/l, pH 8,5) gelöst und mit 50 µl einer 0,15 molaren Trinatriumcitratdihydratlösung versetzt. Nach Zugabe von Pertechnetatlösung (0,4-0,9 mCi) aus einem Mo-99/Tc-99m-Generator gibt man 5 µl einer 0,2 molaren Zinn-(II)-chloriddihydratlösung hinzu und inkubiert 10 min bei Raumtemperatur. Die Markierungsausbeute wird mittels analytischer HPLC bestimmt und ist größer 97%.
c) In vivo und in vitro Anreicherung des Tc-99m-Komplex von N-(Mercaptoacetyl)-Gly-Gly-Asp-(D-Trp)-Leu-Asp-Ile-Ile-Trp in WHHL-Kaninchen.
Die in vivo und in vitro Anreicherung des Tc-99m-Komplex von N-(Mercaptoacetyl)-Gly-Gly-Asp-(D-Trp)-Leu-Asp-Ile-Ile-Trp in WHHL-Kaninchen wurde wie in Beispiel 1 c) beschrieben durchgeführt. Die Ergebnisse sind in Tabelle 1 zusammengefaßt.

Fig. 1 zeigt ein linkslaterales Szintigramm eines WHHL-Kaninchens (A) bzw. eines Neuseeländer Kontrollkaninchens (B) 0,5 h nach i. v. Applikation von 74 MBq Tc-99m-Asp-Gly-Gly-Cys-Gly-Cys-Phe-(D-Trp)-Leu-Asp-Ile-Ile-Trp (vgl. Beispiel 3 c). Aktivitätsanreicherungen im Bereich des Aortenbogens, der linken a. carotis sowie in Bereichen der abdominalen Aorta sind in WHHL-Kaninchen (A) im Gegensatz zu Kontrollkaninchen (B) in vivo sichtbar.

Fig. 2 zeigt ein anteriores Szintigramm eines Neuseeländer Kaninchens dessen rechte a. carotis vier Wochen zuvor mit Hilfe eines Fogarty-Katheters denudiert wurde (A) 0,5 h nach i.v. Applikation von 74 MBq Tc-99m-Asp-Gly-Gly-Cys-Gly-Cys-(D-Trp)-Leu-Asp-Ile-Ile-Trp, sowie ein Autoradiogramm (B1) und Sudan-III-Färbung (B2) der 5 h nach i.v. Applikation herauspräparierten thorokalen Aorta, der denudierten und der unbehandelten a. carotis (vgl. Beispiel 3 c). Die Reihenfolge der herauspräparierten Gefäße bei B1 und B2 jeweils von links nach rechts ist: thorakale Aorta, rechte denudierte a. carotis und linke unbehandelte a. carotis.

Die stark atherosklerotische rechte a. carotis konnte in vivo detektiert werden (A). Die in vitro Untersuchungen (B) bestätigen die gute Korrelation zwischen hoher und selektiver Akkumulation des Radiopharmakons in der denudierten rechten a. carotis im Vergleich zur unbehandelten linken a. carotis.

## Patentansprüche

1. Verbindungen, nämlich
Tc-99m-Komplex von Asp-Gly-Gly-Cys-Gly-Cys-His-Leu-Asp-Ile-Ile-Trp,
Tc-99m-Komplex von Asp-Gly-Gly-Cys-Gly-Cys-(D-Trp)-Leu-Asp-Ile-Ile-Trp,
Tc-99m-Komplex von Asp-Gly-Gly-Cys-Gly-Cys-Phe-(D-Trp)-Leu-Asp-Ile-Ile-Trp,
Tc-99m-Komplex von N-(4-carboxy-1-oxo-3-thia-but-1-yl)-Cys-Phe-(D-Trp)-Leu-Asp-Ile-Ile-Trp,
Tc-99m-Komplex von N-(4-carboxy-1-oxo-3-thia-but-1-yl)-Cys-(D-Trp)-Leu-Asp-Ile-Ile-Trp,
Tc-99m-Komplex von N-(4-carboxy-1-oxo-3-thia-but-1-yl)-Cys-His-Leu-Asp-Ile-Ile-Trp,
Tc-99m-Komplex von N-(Mercaptoacetyl)-Gly-Gly-Asp-Phe-(D-Trp)-Leu-Asp-Ile-Ile-Trp und
Tc-99m-Komplex von N-(Mercaptoacetyl)-Gly-Gly-Asp-(D-Trp)-Leu-Asp-Ile-Ile-Trp.

2. Verbindungen, nämlich
Re-Komplex von Asp-Gly-Gly-Cys-Gly-Cys-His-Leu-Asp-Ile-Ile-Trp,
Re-Komplex von Asp-Gly-Gly-Cys-Gly-Cys-(D-Trp)-Leu-Asp-Ile-Ile-Trp,
Re-Komplex von Asp-Gly-Gly-Cys-Gly-Cys-Phe-(D-Trp)-Leu-Asp-Ile-Ile-Trp,
Re-Komplex N-(4-carboxy-1-oxo-3-thia-but-1-yl)-Cys-Phe-(D-Trp)-Leu-Asp-Ile-Ile-Trp,
Re-Komplex von N-(4-carboxy-1-oxo-3-thia-but-1-yl)-Cys-(D-Trp)-Leu-Asp-Ile-Ile-Trp,
Re-Komplex von N-(4-carboxy-1-oxo-3-thia-but-1-yl)-Cys-His-Leu-Asp-Ile-Ile-Trp,
Re-Komplex von N-(Mercaptoacetyl)-Gly-Gly-Asp-Phe-(D-Trp)-Leu-Asp-Ile-Ile-Trp und
Re-Komplex von N-(Mercaptoacetyl)-Gly-GIy-Asp-(D-Trp)-Leu-Asp-Ile-Ile-Trp.

3. Verbindungen, nämlich
Asp-Gly-Gly-Cys-Gly-Cys-His-Leu-Asp-Ile-Ile-Trp,
Asp-Gly-Gly-Cys-Gly-Cys-(D-Trp)-Leu-Asp-Ile-Ile-Trp,
Asp-Gly-Gly-Cys-Gly-Cys-Phe-(D-Trp)-Leu-Asp-Ile-Ile-Trp,
N-(4-carboxy-1-oxo-3-thia-but-1-yl)-Cys-Phe-(D-Trp)-Leu-Asp-Ile-Ile-Trp,
N-(4-carboxy-1-oxo-3-thia-but-1-yl)-Cys-(D-Trp)-Leu-Asp-Ile-Ile-Trp,
N-(4-carboxy-1-oxo-3-thia-but-1-yl)-Cys-His-Leu-Asp-Ile-Ile-Trp,
N-(Mercaptoacetyl)-Gly-Gly-Asp-Phe-(D-Trp)-Leu-Asp-Ile-Ile-Trp und
N-(Mercaptoacetyl)-Gly-Gly-Asp-(D-Trp)-Leu-Asp-Ile-Ile-Trp.

4. Diagnostisches Mittel, **dadurch gekennzeichnet, daß** es eine Verbindung nach einem der Ansprüche 1 bis 3 und gegebenenfalls geeignete Hilfs- und Trägerstoffe enthält.

## Claims

1. Compounds, namely
Tc-99m-complex of Asp-Gly-Gly-Cys-Gly-Cys-His-Leu-Asp-Ile-Ile-Trp,
Tc-99m-complex of Asp-Gly-Gly-Cys-Gly-Cys-(D-Trp)-Leu-Asp-Ile-Ile-Trp,
Tc-99m-complex of Asp-Gly-Gly-Cys-Gly-Cys-Phe-(D-Trp)-Leu-Asp-Ile-Ile-Trp,
Tc-99m-complex of N-(4-carboxy-1-oxo-3-thia-but-1-yl)-Cys-Phe-(D-Trp)-Leu-Asp-Ile-Ile-Trp,
Tc-99m-complex of N-(4-carboxy-1-oxo-3-thia-but-1-yl)-Cys-(D-Trp)-Leu-Asp-Ile-Ile-Trp ,
Tc-99m-complex of N-(4-carboxy-1-oxo-3-thia-but-1-yl)-Cys-His-Leu-Asp-Ile-Ile-Trp,
Tc-99m-complex of N-(mercaptoacetyl)-Gly-Gly-Asp-Phe-(D-Trp)-Leu-Asp-Ile-Ile-Trp and
Tc-99m-complex of N-(mercaptoacetyl)-Gly-Gly-Asp-(D-Trp)-Leu-Asp-Ile-Ile-Trp.

2. Compounds, namely
Re-complex of Asp-Gly-Gly-Cys-Gly-Cys-His-Leu-Asp-Ile-Ile-Trp,
Re-complex of Asp-Gly-Gly-Cys-Gly-Cys-(D-Trp)-Leu-Asp-Ile-Ile-Trp,
Re-complex of Asp-Gly-Gly-Cys-Gly-Cys-Phe-(D-Trp)-Leu-Asp-Ile-Ile-Trp,
Re-complex of N-(4-carboxy-1-oxo-3-thia-but-1-yl)-Cys-Phe-(D-Trp)-Leu-Asp-Ile-Ile-Trp,
Re-complex of N-(4-carboxy-1-oxo-3-thia-but-1-yl)-Cys-(D-Trp)-Leu-Asp-Ile-Ile-Trp,
Re-complex of N-(4-carboxy-1-oxo-3-thia-but-1-yl)-Cys-His-Leu-Asp-Ile-Ile-Trp,
Re-complex of N-(mercaptoacetyl)-Gly-Gly-Asp-Phe-(D-Trp)-Leu-Asp-Ile-Ile-Trp and
Re-complex of N-(mercaptoacetyl)-Gly-Gly-Asp-(D-Trp)-Leu-Asp-Ile-Ile-Trp.

3. Compounds, namely
Asp-Gly-Gly-Cys-Gly-Cys-His-Leu-Asp-Ile-Ile-Trp,
Asp-Gly-Gly-Cys-Gly-Cys-(D-Trp)-Leu-Asp-Ile-Ile-Trp,
Asp-Gly-Gly-Cys-Gly-Cys-Phe-(D-Trp)-Leu-Asp-Ile-Ile-Trp,
N-(4-carboxy-1-oxo-3-thia-but-1-yl)-Cys-Phe-(D-Trp)-Leu-Asp-Ile-Ile-Trp,
N-(4-carboxy-1-oxo-3-thia-but-1-yl)-Cys-(D-Trp)-Leu-Asp-Ile-Ile-Trp,
N-(4-carboxy-1-oxo-3-thia-but-l-yl)-Cys-His-Leu-Asp-Ile-Ile-Trp,
N-(mercaptoacetyl)-Gly-Gly-Asp-Phe-(D-Trp)-Leu-Asp-Ile-Ile-Trp and
N-(mercaptoacetyl)-Gly-Gly-Asp-(D-Trp)-Leu-Asp-Ile-Ile-Trp.

4. Diagnostic agent, **characterized in that** it contains a compound according to any of Claims 1 to 3 and, optionally, suitable auxiliaries and excipients.

## Revendications

1. Composés, à savoir
complexe Tc-99m de Asp-Gly-Gly-Cys-Gly-Cys-His-Leu-Asp-Ile-Ile-Trp,
complexe Tc-99m de Asp-Gly-Gly-Cys-Gly-Cys-(D-Trp)-Leu-Asp-Ile-Ile-Trp,
complexe Tc-99m de Asp-Gly-Gly-Cys-Gly-Cys-Phe-(D-Trp)-Leu-Asp-Ile-Ile-Trp,
complexe Tc-99m de N-(4-carboxy-1-oxo-3-thia-but-1-yl)-Cys-Phe-(D-Trp)-Leu-Asp-Ile-Ile-Trp,
complexe Tc-99m de N-(4-carboxy-1-oxo-3-thia-but-1-yl)-Cys-(D-Trp)-Leu-Asp-Ile-Ile-Trp,
complexe Tc-99m de N-(4-carboxy-1-oxo-3-thia-but-1-yl)-Cys-His-Leu-Asp-Ile-Ile-Trp,
complexe Tc-99m de N-(mercaptoacétyl)-Gly-Gly-Asp-Phe-(D-Trp)-Leu-Asp-Ile-Ile-Trp et
complexe Tc-99m de N-(mercaptoacétyl)-Gly-Gly-Asp-(D-Trp)-Leu-Asp-Ile-Ile-Trp.

2. Composés, à savoir
complexe Re de Asp-Gly-Gly-Cys-Gly-Cys-His-Leu-Asp-Ile-Ile-Trp,
complexe Re de Asp-Gly-Gly-Cys-Gly-Cys-(D-Trp)-Leu-Asp-Ile-Ile-Trp,
complexe Re de Asp-Gly-Gly-Cys-Gly-Cys-Phe-(D-Trp)-Leu-Asp-Ile-Ile-Trp,
complexe Re de N-(4-carboxy-1-oxo-3-thia-but-1-yl)-Cys-Phe-(D-Trp)-Leu-Asp-Ile-Ile-Trp,
complexe Re de N-(4-carboxy-1-oxo-3-thia-but-1-yl)-Cys-(D-Trp)-Leu-Asp-Ile-Ile-Trp,
complexe Re de N-(4-carboxy-1-oxo-3-thia-but-1-yl)-Cys-His-Leu-Asp-Ile-Ile-Trp,
complexe Re de N-(mercaptoacétyl)-Gly-Gly-Asp-Phe-(D-Trp)-Leu-Asp-Ile-Ile-Trp et
complexe Re de N-(mercaptoacétyl)-Gly-Gly-Asp-(D-Trp)-Leu-Asp-Ile-Ile-Trp.

3. Composés, à savoir
Asp-Gly-Gly-Cys-Gly-Cys-His-Leu-Asp-Ile-Ile-Trp,
Asp-Gly-Gly-Cys-Gly-Cys-(D-Trp)-Leu-Asp-Ile-Ile-Trp,
Asp-Gly-Gly-Cys-Gly-Cys-Phe-(D-Trp)-Leu-Asp-Ile-Ile-Trp,
N-(4-carboxy-1-oxo-3-thia-but-1-yl)-Cys-Phe-(D-Trp)-Leu-Asp-Ile-Ile-Trp,
N-(4-carboxy-1-oxo-3-thia-but-1-yl)-Cys-(D-Trp)-Leu-Asp-Ile-Ile-Trp,
N-(4-carboxy-1-oxo-3-thia-but-1-yl)-Cys-His-Leu-Asp-Ile-Ile-Trp,
N-(mercaptoacétyl)-Gly-Gly-Asp-Phe-(D-Trp)-Leu-Asp-Ile-Ile-Trp et
N-(mercaptoacétyl)-Gly-Gly-Asp-(D-Trp)-Leu-Asp-Ile-Ile-Trp.

4. Moyen de diagnostic **caractérisé en ce qu'**il comprend un composé selon une des revendications 1 à 3 et, le cas échéant, des substances auxiliaires et supports idoines.
